# EUROPEAN PATENT SPECIFICATION

(11) **EP 2 646 015 B1**
(45) Date of publication and mention of the grant of the patent: **22.04.2020**
(21) Application number: 11845087.3
(22) Date of filing: 30.11.2011
(51) Int. Cl.: A61K 31/221, A61K 31/4439, A61K 47/18, A61P 1/04

(54) **ACTIVE ENANTIOMER OF DODECYL 2-(N,N-DIMETHYLAMINO)-PROPIONATE**
AKTIVES ENANTIOMER AUS DODECYL-2-(N,N-DIMETHYLAMINO)-PROPIONAT
ÉNANTIOMÈRE ACTIF DU 2-(N,N-DIMÉTHYLAMINO)-PROPIONATE DODÉCYLIQUE

(30) Priority: 02.12.2010 US 418996 P
(43) Date of publication of application: 09.10.2013
(73) Proprietor: Ferring International Center S.A., 1162 Saint-Prex (CH)
(72) Inventor: DAMAJ, Bassam B., San Diego California 92127 (US); MARTIN, Richard, San Diego California 92130 (US)
(74) Representative: Hoyng Rokh Monegier LLP
(86) International application number: PCT/US2011/062579
(87) International publication number: WO 2012/075107

(56) References cited:
- US-A- 6 118 020
- US-A- 6 118 020
- JAKUB NOVOTN PRG A[1/2] ET AL: "Dimethylamino Acid Esters as Biodegradable and Reversible Transdermal Permeation Enhancers: Effects of Linking Chain Length, Chirality and Polyfluorination", PHARMACEUTICAL RESEARCH, KLUWER ACADEMIC PUBLISHERS-PLENUM PUBLISHERS, NL, vol. 26, no. 4, 14 November 2008 (2008-11-14), pages 811-821, XP019686119, ISSN: 1573-904X
- HU.: 'TRANSDERMAL AND TRANSBUCCAL DRUG DELIVERY: ENHANCMENT USING IONTOPHORESIS AND CHEMICAL ENHANCERS.' A DISSERTATION SUBMITTED TO THE GRADUATE SCHOOL-NEW BRUNSWICK RUTGERS October 2010, THE STATE UNIVERSITY OF NEW JERSEY, XP055136368

## Description

### Technical Field

This invention relates to enantiomers of dodecyl 2-(N,N-dimethylamino)-propionate (DDAIP), and in particular the R-enantiomer thereof.

### Background of the Invention

The advantages of transdermal drug delivery over other methods of drug administration are well recognized. Working alone, most drugs do not sufficiently permeate the skin or other membranes to provide therapeutic levels of drug delivery. The skin, especially the outer layer (stratum corneum), provides a formidable barrier to the penetration of most substances. To overcome the skin's natural protective barrier, topical drug formulations typically include a skin penetration enhancer. Skin penetration enhancers also may be referred to as absorption enhancers, accelerants, adjuvants, solubilizers, sorption promoters, etc. Whatever the name, such agents serve to improve drug absorption across the skin. Ideal penetration enhancers not only increase drug flux across the skin, but do so without irritating, sensitizing, or damaging skin. Furthermore, ideal penetration enhancers should not adversely affect the stability of the active drug, the physical stability of the dosage form (e.g. cream or gel), or the cosmetic quality of the topical composition.

A wide variety of compounds have been evaluated as to their effectiveness in enhancing the rate of penetration of drugs through the skin, as well as enhancing penetration through other biological membranes (e.g., the stomach lining, the small intestine, the colon, finger nail, toe nail, etc.). See, for example, Büyüktimkin et al., Chemical Means of Transdermal Drug Permeation Enhancement in Transdermal and Topical Drug Delivery Systems, Ghosh T.K., Pfister W.R., Yum S.I. (Eds.), Interpharm Press Inc., Buffalo Grove, IL (1997), which surveys the use and testing of various skin penetration enhancers.

Of the many groups of compounds being evaluated, several racemic alkyl (N,N-disubstituted amino alkanoate) esters have shown promise as penetration enhancers. Of the alkyl (N,N-disubstituted amino alkanoate) esters, the racemic (R,S) form of dodecyl 2-(N,N dimethylamino)-propionate (DDAIP) has shown particular promise because of its confirmed biodegradability. For a discussion of the penetration enhancing properties of DDAIP see Büyüktimkin et al., Alkyl N,N-Disubstituted-Amino Acetates in Percutaneous Penetration Enhancers, Maibach H. I. and Smith H. E. (eds.), CRC Press, Inc., Boca Raton, F.L. (1995).

The racemic form of DDAIP, which may also be referred to as dodecyl 2-methyl-2-(N,N-dimethyl amino) acetate, which has the following chemical formula, 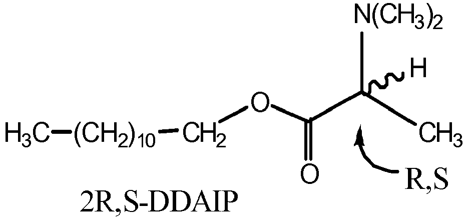 is a liquid at room temperature, and is an effective skin penetration enhancer for a wide variety of medicaments. Racemic DDAIP is not soluble in water, but is miscible with most organic solvents. Table I, below, contains a list of other reported attributes of racemic DDAIP.

**Table I**

| **Physical Properties Of Racemic DDAIP** | |
|---|---|
| Molecular Weight | 285.47 |
| CAS Number | 149196-89-4 |
| Physical form | Clear colorless liquid |
| Freezing point | -17.5 ° C |
| Boiling point | 142 - 144 ° C/0.1 mmHG |
| Viscosity | 7.32 centiStokes at 23 ° C |
| Refractive Index (nD) | 1.4435 at 24.5 ° C |
| Specific gravity (D₂₃₎ | 0.85 |

Salts of racemic DDAIP, including crystalline salts, also have been prepared and are effective as penetration enhancers. U.S. Patent No. 6, 118,020 describes the preparation and evaluation of some particularly preferred crystalline salts of DDAIP.

Enantiomerically enriched forms of DDAIP (i.e., DDAIP in predominately the S or predominately the R configuration) have heretofore been evaluated as membrane penetration enhancers in Novotny et al., Pharm. Res. 2009, 26 (4), 811-821 (D1). D1 does not disclose the administration of a solution containing DDAIP and concludes on lack of stereoselectivity in action of DDAIP for the specific drugs tested therein.

### Summary of the Invention

The present invention provides dosage forms according to the claims and containing a enantiomerically enhanced forms of dodecyl 2-(N,N-dimethylamino)-propionate (DDAIP) that are predominately in the 2R-configuration. Mentions of 2S-DDAIP do not belong to the invention but are left for a better understanding of the same. The 2R-DDAIP exhibits enhanced activity *vis-a-vis* facilitating transport of materials (e.g., a pharmaceutically active compound comprising lansoprazole) across a biological membrane or tissue, compared to the same amount of racemic DDAIP or S-DDAIP of similar or the same enantiomeric purity. The structural formulas for 2R-DDAIP and 2S-DDAIP are provided below.

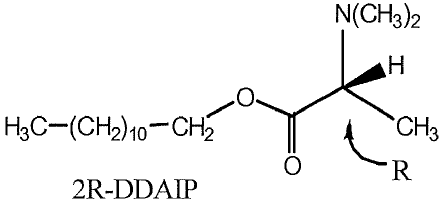

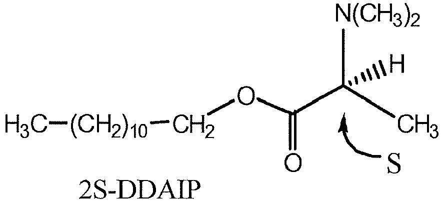

For convenience, a DDAIP that is predominately in the 2R enantiomeric configuration will be referred to herein as "R-DDAIP" regardless of the level of enantiomeric purity ofthe material. Similarly, as used herein, "S-DDAIP" refers to a DDAIP that is enriched in the 2S-enantiomer, regardless ofthe enantiomeric purity ofthe material.

In a preferred embodiment, the R-DDAIP has enantiomeric purities ofat least about 70 %, more preferably at least about 80 %.

The **R-DDAIP** can be in the free base form, or a salt form (e.g., a crystalline salt). The salts of **DDAIP** according to the present invention include inorganic acid addition salts such as the hydrochloric, hydrobromic, sulfuric, phosphoric, and nitric acid addition salts, as well as organic acid addition salts such as the acetic, benzoic, salicylic, glycolic, succinic, nicotinic, tartaric, maleic, malic, pamoic, methanesulfonic, cyclohexanesulfamic, picric, and lactic acid addition salts. Preferred crystalline **DDAIP** salts are **DDAIP** hydrogen chloride and **DDAIP** dihydrogen sulfate.

In another aspect, the present invention provides a method of facilitating penetration or transport ofa pharmaceutically active compound through or across a biological membrane or tissue as defined in the claims. The method comprises contacting the membrane or tissue with a dosage form of the invention.

### Brief Description of the Drawings

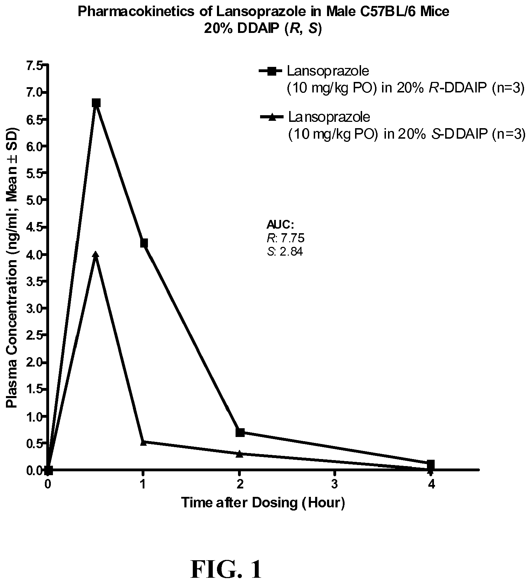

FIGURE 1 provides a comparison graph of the plasma concentration of lansoprazole versus time after dosing, obtained from mice that were orally administered an aqueous solution comprising about 10 milligrams-per-milliliter (mg/ml) lansoprazole and about 20% by weight R-DDAIP or S-DDAIP, at a lansoprazole dosage of about 10 milligrams-per-kilogram (mg/kg).

### Detailed Description of Preferred Embodiments

While this invention is susceptible to embodiments in many different forms, preferred embodiments of the invention are described below. It should be understood, however, that the present disclosure is to be considered as a exemplification ofthe principles of the invention and is not intended to limit the invention to the specific embodiments illustrated.

In one aspect, the present disclosure provides a method of facilitating transport of a pharmaceutically active compound through a biological membrane or tissue The method comprises contacting the membrane or tissue with a dosage form of the invention.

Preferably, the 2R-dodecyl 2-(N,N-dimethylamino)-propionate has an enantiomeric purity of at least about 80 %, even more preferably at least about 90, 95, or 98 %. As used herein, the term "enantiomeric purity" refers to the mole percentage ofthe specified enantiomer in the material, as determined by any suitable method (e.g., chiral high performance liquid chromatography, optical rotation, and the like).

In a preferred embodiment, the facilitating comprises increasing the rate of oral uptake ofthe pharmaceutically active compound as defined in the claims into the blood stream of a mammal when a solution of the pharmaceutically active compound and the 2R-dodecyl 2-(N,N-dimethylamino)-propionate is administered to the mammal, as compared to the rate of uptake observed with 2S-dodecyl 2-(N,N-dimethylamino)-propionate at the same dosage level and the same or similar enantiomeric purity.

In the methods of the present disclosure the R-DDAIP can be utilized in the free base form, or as a salt (e.g., a crystalline salt. Preferably, the R-DDAIP and the pharmaceutically active material are co-administered in a solution, preferably an aqueous-based solution.

Racemic DDAIP can be conveniently manufactured by transesterification of ethyl 2-(N,N-dimethylamino) propionate. To this end, ethyl 2-(N,N-dimethylamino) propionate is heated with 1-dodecanol in the presence of a transesterification catalyst.

A wide variety of transesterification catalysts is available for this purpose. Preferred are basic transesterification catalysts such as the alkali metal alkoxides, e.g. sodium methoxide, potassium methoxide, and the like. Other suitable basic transesterification catalysts are n-butyl lithium, potassium cyanide, and the like.

The method for the manufacture of such DDAIP acid addition salts comprises combining DDAIP with a selected acid in the presence of a water-immiscible solvent to form a salt precipitate and then recovering the salt precipitate, from solution. The DDAIP is combined with the selected acid at a controlled temperature in the range of about 10 to about -10 °C. The water-immiscible solvent is preferably an aliphatic hydrocarbon, more preferably hexane.

Crystalline, acid addition salts of dodecyl 2-(N,N-dimethylamino)-propionate (DDAIP), including R-DDAIP, can be inorganic as well as organic. Representative inorganic acid addition salts include the hydrochloric, hydrobromic, sulfuric, phosphoric, nitric acid addition salts of DDAIP, and their solvates. Exemplary organic acid addition salts include acetic, benzoic, salicylic, glycolic, succinic, nicotinic, tartaric, maleic, malic, pamoic, methanesulfonic, cyclohexanesulfamic, picric, and lactic acid addition salts, as well as their respective solvates.

The preparation of alkyl-2-(N,N-disubstituted amino)-alkanoates such as DDAIP is well known in the art, see e.g., U.S. Patent No. 4,980,378 to Wong et al.

The R-DDAIP and S-DDAIP can be prepared by any suitable method known in the art. For example, the enantiomers can be prepared from racemic DDAIP by chiral resolution methods, which are well known in the art. Preferably, the R-DDAIP is prepared from a suitably N-protected D-alanine (e.g., N-benzyloxycarbonyl-protected D-alanine) by esterification with dodecanol, removal of the protecting group, and reductive methylation of the amino group (e.g., by hydrogenation in the presence of formaldehyde). S-DDAIP can be similarly prepared from L-alanine. The choice of a suitable protecting group is well within the ordinary level of skill in the art, and generally will be determined by the conditions used in the esterification reaction (i.e., the protecting group should remain in place during esterification) and should be removable under conditions that will not affect the ester or racemize the product.

Certain aspects of the present invention are illustrated by the following non-limiting examples.

### Example 1: Preparation of R-DDAIP and S-DDAIP

The R and S enantiomers of DDAIP were synthesized as follows. In the case of the S enantiomer, the synthesis utilized N-benzyloxycarbonyl protected L-alanine, while the R enantiomer was prepared from the corresponding protected D-alanine. The benzyloxycarbonyl alanine materials were esterified with dodecanol in toluene, with azeotropic removal of water under reflux. A few drops of concentrated sulphuric acid added to drive the esterification reaction to completion. The benzyloxycarbonyl protecting group was removed from each material by hydrogenation to provide a good yield of the primary amino compound. Once the deprotection was complete, 37% formaldehyde was added, and the hydrogenation was continued until reductive methylation of the amino group was complete. The crude products were purified by column chromatography and further purified by crystallization of the hydrochloride salts in acetone. The free-base forms of the R-DDAIP and the S-DDAIP were obtained by adding sodium bicarbonate solution to the respective salts, and extracting the free-base into tertiary butyl methyl ether. The solvent was then removed to provide the product R-DDAIP or S-DDAIP, as the case may be. The purity of the products, by gas chromatographic analysis, was greater than about 98 % in each case. The L-alanine and D-alanine used in the procedure described above were essentially >99 percent enantiomerically pure. Accordingly, the resulting S-DDAIP and R-DDAIP are believed to be at least greater than 80 percent enantiomerically pure, most likely greater than 99 percent enantiomerically pure, as well, since the conditions used in their synthesis are not known to cause racimization.

### Example 2: Enhancement of Oral Absorption of Lansoprazole

Lansoprazole (CAS No. 103577-45-3) is a well known proton-pump inhibitor utilized in a number of prescription and over-the-counter mediations for treating the symptoms of heartburn and gastric reflux. Male C57BL/6J mice (Jackson Labs, USA) having a weight of about 25 to about 30 g were dosed by oral gavage (PO) with freshly prepared solutions of about 2 mg/ml of lansoprazole and about 20 % by weight of either R-DDAIP or S-DDAIP free-base, in water. The solutions were administered in the morning after feeding. Two groups of mice (n=3) were orally dosed once with the lansoprazole solutions at a lansoprazole dosage of about 10 mg/kg. The gavage volume was about 5 ml/kg for all groups. Blood samples were collected by cheek-bleed at the 0.5-hour, 1-hour, and 2-hour time-points, with the final 4-hour sample obtained by cardiac puncture following isoflurane euthanasia. All blood samples were collected into tubes containing K₂EDTA and processed to plasma in a 4 °C centrifuge within about 10 minutes of collection. Plasma samples were stored at about -80 °C until quantitation by LC/MS/MS analysis. The analytical data are summarized in graphic format in FIG. 1. The results in FIG. 1 demonstrate that R-DDAIP was unexpectedly about 2.7 times more effective at enhancing oral uptake of lansoprazole into the blood stream compared to S-DDAIP, as determined by the integrated area under the respective plasma concentration-versus-time curves (AUC).

The foregoing is intended to be illustrative of the present invention, but not limiting.

## Claims

1. A dosage form comprising a pharmaceutically active compound and 2R-dodecyl 2-(N,N-dimethylamino)-propionate or a salt thereof, wherein the 2R-dodecyl 2-(N,N-dimethylamino)-propionate has an enantiomeric purity of at least about 70 %, wherein the dosage form is a solution, wherein the pharmaceutically active compound comprises lansoprazole.

2. The dosage form of claim 1 wherein the 2R-dodecyl 2-(N,N-dimethylamino)-propionate of claim 1 has an enantiomeric purity of at least about 80 %.

3. The dosage form of claim 1 wherein the 2R-dodecyl 2-(N,N-dimethylamino)-propionate of claim 1 has an enantiomeric purity of at least about 90 %.

4. The dosage form of claim 1 wherein the 2R-dodecyl 2-(N,N-dimethylamino)-propionate of claim 1 has an enantiomeric purity of at least about 98 %.

5. The dosage form of claim 1 wherein the salt is selected from the group consisting of hydrochloric, hydrobromic, sulfuric, phosphoric, and nitric acid addition salts.

6. The dosage form of claim 1 wherein the salt is selected from the group consisting of acetic, benzoic, salicylic, glycolic, succinic, nicotinic, tartaric, maleic, malic, pamoic, methanesulfonic, cyclohexanesulfamic, picric, and lactic acid addition salts.

7. The dosage form of claim 1, which is for oral administration.

## Patentansprüche

1. Darreichungsform, umfassend eine pharmazeutisch aktive Verbindung und 2R-Dodecyl- 2-(N,N-dimethylamino)-propionat oder ein Salz davon, wobei das 2R-Dodecyl- 2-(N,N-dimethylamino)-propionat eine enantiomere Reinheit von mindestens etwa 70 % aufweist, wobei die Darreichungsform eine Lösung ist, wobei die pharmazeutisch aktive Verbindung Lansoprazol umfasst.

2. Darreichungsform nach Anspruch 1, wobei das 2R-Dodecyl- 2-(N,N-dimethylamino)-propionat nach Anspruch 1 eine enantiomere Reinheit von mindestens etwa 80 % aufweist.

3. Darreichungsform nach Anspruch 1, wobei das 2R-Dodecyl- 2-(N,N-dimethylamino)-propionat nach Anspruch 1 eine enantiomere Reinheit von mindestens etwa 90 % aufweist.

4. Darreichungsform nach Anspruch 1, wobei das 2R-Dodecyl- 2-(N,N-dimethylamino)-propionat nach Anspruch 1 eine enantiomere Reinheit von mindestens etwa 98 % aufweist.

5. Darreichungsform nach Anspruch 1, wobei das Salz aus der Gruppe bestehend aus Salzsäure-, Bromsäure-, Schwefelsäure-, Phosphorsäure- und Salpetersäureadditionssalzen ausgewählt ist.

6. Darreichungsform nach Anspruch 1, wobei das Salz aus der Gruppe bestehend aus Essigsäure-, Benzoesäure-, Salicylsäure-, Glykolsäure-, Bernsteinsäure-, Nikotinsäure-, Weinsäure-, Maleinsäure-, Äpfelsäure-, Pamoasäure-, Methansulfonsäure-, Cyclohexansulfamsäure-, Pikrinsäure- und Milchsäureadditionssalzen ausgewählt ist.

7. Darreichungsform nach Anspruch 1, die zur oralen Verabreichung bestimmt ist.

## Revendications

1. Forme posologique comprenant un composé pharmaceutiquement actif et du 2R-dodécyl 2-(N,N-diméthylamino)-propionate ou un sel de celui-ci, dans laquelle le 2R-dodécyl 2-(N,N-diméthylamino)-propionate a une pureté énantiomérique d'au moins environ 70 %, dans laquelle la forme posologique est une solution, dans laquelle le composé pharmaceutiquement actif comprend du lansoprazole.

2. Forme posologique selon la revendication 1, dans laquelle le 2R-dodécyl 2-(N,N-diméthylamino)-propionate de la revendication 1 a une pureté énantiomérique d'au moins environ 80 %.

3. Forme posologique selon la revendication 1, dans laquelle le 2R-dodécyl 2-(N,N-diméthylamino)-propionate de la revendication 1 a une pureté énantiomérique d'au moins environ 90 %.

4. Forme posologique selon la revendication 1, dans laquelle le 2R-dodécyl 2-(N,N-diméthylamino)-propionate de la revendication 1 a une pureté énantiomérique d'au moins environ 98 %.

5. Forme posologique selon la revendication 1, dans laquelle le sel est choisi dans le groupe consistant en les sels d'addition avec les acides chlorhydrique, bromhydrique, sulfurique, phosphorique et nitrique.

6. Forme posologique selon la revendication 1, dans laquelle le sel est choisi dans le groupe consistant en les sels d'addition avec les acides acétique, benzoïque, salicylique, glycolique, succinique, nicotinique, tartrique, maléique, malique, pamoïque, méthanesulfonique, cyclohexanesulfamique, picrique et lactique.

7. Forme posologique selon la revendication 1, destinée à une administration par voie orale.
